# EUROPEAN PATENT APPLICATION

(11) **EP 3 533 386 A1**
(43) Date of publication of application: **04.09.2019**
(21) Application number: 18159049.8
(22) Date of filing: 28.02.2018
(51) Int. Cl.: A61B 5/00, A61B 5/0215, G01L 9/00

(54) **PRESSURE SENSING WITH CAPACITIVE PRESSURE SENSOR**

(71) Applicant: Koninklijke Philips N.V., 5656 AE Eindhoven (NL)
(72) Inventor: DIRKSEN, Peter, 5656 AE Eindhoven (NL); MAUCZOK, Ruediger, 5656 AE Eindhoven (NL); TIMMERMANS, Petrus Henricus Maria, 5656 AE Eindhoven (NL)
(74) Representative: de Haan, Poul Erik

(57) **Abstract**

A capacitive pressure sensor (10) is disclosed comprising a membrane (21) including a second electrode (23) spatially separated by a cavity (20) from a substrate (11) including a first electrode (13) opposing the second electrode; and a central pillar (22) extending from the membrane to the substrate such that the cavity is an annular cavity enveloping said central pillar. Also disclosed is an invasive medical instrument comprising such a capacitive pressure sensor and a method of manufacturing such a capacitive pressure sensor.

## Description

### FIELD OF THE INVENTION

The present invention relates to a capacitive pressure sensor comprising a membrane spatially separated from a substrate by a cavity, a first electrode in or on said substrate and a second electrode in or on said membrane opposing the first electrode.

The present invention further relates to a medical instrument for insertion into a patient such as a catheter including such a capacitive pressure sensor.

The present invention still further relates to a method for manufacturing such a capacitive pressure sensor.

### BACKGROUND OF THE INVENTION

The role of medical diagnostics in modern society is of an ever increasing importance. For instance, sedentary lifestyles, diet and lifestyle choices such as consumption of alcohol and smoking put an increased pressure on healthcare providers to ensure people stay healthy throughout their life. This for example involves managing the risk of cardiovascular diseases such as angina, cardiac infarction and stroke in such individuals.

To this end, medical practitioners may use a range of diagnostic instruments such as scanning instruments (MRI, CT, and so on) or (minimally) invasive medical instruments, e.g. catheters or the like to diagnose and localize certain anomalies in the cardiovascular system of a patient in order to address such anomalies. The use of minimally invasive medical instruments is particularly advantageous where a location of the anomaly such as a stenosis for example needs to be determined accurately, such that a medical procedure such as the placement of a stent or the like to correct the anomaly is performed in the correct location. As is well known per se, such a medical procedure may be performed with the same invasive medical instrument or with a separate invasive medical instrument.

In order to pinpoint the location of the anomaly, the medical instrument may be equipped with a sensor that directly or indirectly senses the presence of the anomaly within the cardiovascular system of the patient, e.g. in a vein or artery. For example, in case of a stenosis, the sensor may include one or more pressure sensors distributed along the elongation direction of the medical instrument at the instrument tip that sense the blood pressure at a particular location within the cardiovascular system of the patient. A commonly used indicator for a lesion such as a stenosis is the fractional flow reserve (FFR), which may be approximated as: FFR ≈ P_{D}/P_{A}, in which P_{D} is the pressure distal to the lesion and P_{A} is the pressure proximal to the lesion.

A particular challenge when providing one or more pressure sensors on such a medical instrument for insertion into a patient is that the pressure sensor has to be very small in order to fit on the medical instrument and to not impede insertion and passage of the medical instrument through the cardiovascular system of the patient. This typically requires the dimensions (e.g. the diameter) of the pressure sensor to be well below 1 mm.

A particularly promising class of pressure sensors for such applications includes capacitive micromachined pressure sensors such as capacitive micromachined pressure sensors. Such pressure sensors typically comprise a flexible membrane suspended over a cavity, with the membrane comprising an electrode opposing a further electrode at the cavity floor, i.e. the substrate. The pressure on the membrane defines the degree of deformation of the membrane towards the substrate, which alters the (average) distance between the opposing electrodes. This alters the capacitance of the capacitor formed by the spatially separated opposing electrodes, which capacitance can be measured by providing a potential difference across the electrodes.

An example of such a capacitive pressure sensor is disclosed in WO2013/072803 A1 in which a pre-collapsed capacitive micro-machined transducer cell comprising a substrate comprising a first electrode and a membrane comprising a second electrode is disclosed. The cell has an outer region where the membrane is mounted to the substrate and an inner region inside or surrounded by the outer region. The membrane is collapsed to the substrate in a first collapsed annular-shaped region located within the inner region. By operating the capacitive micro-machined transducer cell in such a collapsed mode, the sensitivity of the cell is improved compared to pressure sensor cells that are operated in a non-collapsed mode, such as the ultrasound pressure sensor cell disclosed in US 2011/0198966 A1.

However, it has been found that for at least some capacitive micro-machined pressure sensor cell designs operated in a collapse mode, the pressure readings obtained with such cells are prone to hysteresis effects, which compromises the accuracy of the pressure readings. Such hysteresis effects are expressed by the sensor given different readings for the same blood pressure depending on whether the blood pressure is increasing or decreasing, i.e. during different phases of the cardiac cycle. This is of course highly unwanted when trying to accurately locate an anomaly within the cardiovascular system of the patient.

### SUMMARY OF THE INVENTION

The present invention seeks to provide a capacitive pressure sensor that can be operated with high sensitivity whilst being less prone to exhibit hysteresis and drift effects in its pressure readings.

The present invention further seeks to provide a medical instrument for insertion into a patient comprising such a capacitive pressure sensor.

The present invention yet further seeks to provide a method for manufacturing such a capacitive pressure sensor.

According to an aspect, there is provided a capacitive pressure sensor comprising a membrane including a second electrode spatially separated by a cavity from a substrate including a first electrode opposing the second electrode and a central pillar extending from the membrane to the substrate such that the cavity is an annular cavity enveloping said central pillar . Such a capacitive pressure sensor may be operated in a non-collapse mode. It has been surprisingly found that by anchoring the central region of the membrane to the substrate of the capacitive pressure sensor with a central pillar such that an annular cavity is formed around the central pillar, the capacitive pressure sensor exhibits improved sensitivity compared to a capacitive pressure sensor having a circular cavity operated in a non-collapse mode. In fact, the sensitivity of the capacitive pressure sensor according to embodiments of the present invention is comparable to that of capacitive pressure sensors operated in a (pre-)collapse mode but without exhibiting the hysteresis effects from which such capacitive pressure sensors operated in (pre-)collapse mode typically suffer.

Preferably, the cavity has an inner edge defined by the central pillar and an opposing outer edge, said inner edge and opposing outer edge having substantially the same height as it has been found that for such a geometrical arrangement the capacitive pressure sensor exhibits optimal performance. In this context, the term 'substantially the same height' refers to the fact that the cavity may be formed from the release of a planar portion of a sacrificial material as will be explained in more detail below. As will be understood, the respective heights of the inner and outer edges of the resulting cavity therefore will vary by no more than the limitations in the thickness control of the deposition technique used to deposit such a planar sacrificial layer.

Preferably, the second electrode is a continuous electrode aligned with said first electrode. This may improve the accuracy of the capacitive pressure sensor, in particular where the second electrode is operated as the ground electrode and the first electrode is operated as the sensing electrode, such that the second electrode provides optimal shielding of the first electrode from external electrostatic disturbances. The second electrode alternatively may be an annular electrode aligned with the annular cavity, which provides less effective shielding in such a scenario.

In order for the capacitive pressure sensor to be operable in a non-collapse mode, the membrane typically is dimensioned such that at typical blood pressures part of the membrane does not collapse onto the substrate of the capacitive pressure sensor. For example, the (absolute) collapse pressure P_{collapse} of the capacitive pressure sensor maybe in a range of 1.7 Bar < P_{collapse} < 2.3 bar, with P = 0 bar corresponding to vacuum. If the collapse pressure is lower than 1.7 Bar, collapse during in vivo pressure sensing may occur, whilst if the collapse pressure is higher than 2.3 bar, the sensitivity of the capacitive pressure sensor may be significantly reduced.

To this end, the membrane may have a thickness in a range of 0.8-2.0 micron and a diameter in a range of 20-200 micron. For the avoidance of doubt, the (outer) diameter of the membrane is defined as the distance between two diametrically opposing points on the outer edge of the annular cavity of the capacitive pressure sensor, i.e. the edge of the annular cavity extending from the substrate to the membrane that is distal to the central pillar. At this point it is noted that the collapse pressure P_{collapse} scales according to P_{collapse} ∼ T³ * H/D⁴ in which T is the membrane thickness, H is the height of the annular cavity and D is the membrane diameter, from which it can be seen that the membrane dimensions and annular cavity height may be varied in a coordinated manner in order to maintain a desirable collapse pressure for the capacitive pressure sensor, such that the embodiments of the present invention are not necessarily limited to the disclosed ranges of these parameters. Instead, trade-offs between T, H and D maybe made in order to achieve the desired collapse pressure P_{collapse}, at which the capacitive pressure sensor may be operated in a non-collapsed mode of operation.

In order to optimize sensitivity of the capacitive pressure sensor, the annular cavity has a height (H) in a range of 50-500 nm, preferably in a range of 100-400 nm, wherein the height is defined as the distance from the substrate to the membrane of the capacitive pressure sensor when the membrane is substantially planar.

In some embodiments, the capacitive pressure sensor further comprises a rim surrounding the membrane, said rim having a further thickness larger than the membrane thickness. Such a rim may be formed when the membrane thickness is reduced following the formation of the cavity, i.e. by removal of the sacrificial material, and subsequent sealing of the opening through which the sacrificial material was evacuated from the cavity in order to obtain a stable membrane of minimal thickness. In such a process, the rim is formed by selective removal of the sealing material from over the membrane area only, such that the sealing material outside this area remains and defines the rim. Such a rim may add further structural support to the thin membrane, i.e. may stabilize the membrane.

In a preferred embodiment, the rim is laterally displaced relative to the outer edge of the annular cavity such that the rim is further removed from the central pillar than said outer edge. In other words, a planar annular region is formed between the membrane and the rim that surrounds the membrane. It has surprisingly been found that where the rim is laterally displaced relative to the outer edge of the cavity, drift in the sensitivity of the capacitive pressure sensor over time is greatly reduced. This can be explained by the fact that over time and during use, contamination such as dirt particles and other deposits collects at the inner edge of the rim, which when the rim is aligned with the outer edge of the annular cavity causes such particles to collect on the outer edge of the membrane, thereby altering the membrane characteristics, e.g. membrane resonance frequency. By laterally displacing the rim, a collection area for such contamination is provided between the membrane and the rim, such that the membrane performance is much more stable over time due to such contamination collecting outside the membrane area. In at least some embodiments the rim has a width in a range of 2-5 microns.

The substrate may further comprise a circuit arrangement adapted to process sensor signals generated with the capacitive pressure sensor; and a pair of terminals conductively coupled to the circuit arrangement for receiving power supply wires. The circuit arrangement, e.g. an ASIC or the like may perform some (pre-)processing of the sensor signals, such as digitization and/or amplification such that the capacitive pressure sensor itself can be kept as small as possible. Without such signal processing circuitry, the physical dimensions of the capacitive pressure sensor are limited by the length of wire(s) over which the sensor signals are to be carried to a user console or the like, as signal losses over such wires dictate that the initial signal strength of the signals produced by the capacitive pressure sensor is sufficient such that a processable residual sensor signal is received by the user console after signal losses. Consequently, the inclusion of the signal processing circuit arrangement in the substrate ensures that the sensor signals may be weaker, i.e. that the capacitive pressure sensor may be smaller. To further reduce the form factor of the capacitive sensor signal, the circuit arrangement further may be adapted to modulate the power supply with the processed sensor signal, such that the substrate 11 does not require a separate signal terminal and only needs connecting to two power supply wires.

According to another aspect, there is provided a medical instrument for insertion into a patient having an instrument tip comprising the capacitive pressure sensor according to any of the herein described embodiments as explained above. Such a medical instrument, e.g. a minimally invasive medical instrument, benefits from the improved operational stability and sensitivity of the capacitive pressure sensor and may therefore be used to accurately determine the blood pressure within the cardiovascular system of a patient, e.g. to determine an anomaly or lesion such as a stenosis. In a first set of embodiments, such an anomaly may be determined by performing a first measurement with the capacitive pressure sensor in an initial location, displacing the capacitive pressure sensor (e.g. by altering the degree of insertion of the medical instrument into the patient) and performing a further measurement with the capacitive pressure sensor in the altered location, e.g. to determine the FFR from the respectively sensed pressures in the initial and altered locations.

Alternatively, the medical instrument may comprise a plurality of said capacitive pressure sensors distributed along an elongation direction of the invasive medical instrument such that such pressure measurements in different locations may be performed simultaneously, i.e. utilizing capacitive pressure sensors in different locations along the tip of the medical instrument.

According to yet another aspect, there is provided a method for manufacturing a capacitive pressure sensor, comprising providing a substrate; forming a first electrode on said substrate; depositing an annulus of a sacrificial material on the substrate; forming a second electrode on said annulus; depositing a membrane material over the annulus, thereby filling the central cavity of the annulus; opening the membrane material to expose said annulus; removing the sacrificial material through said opening to form an annular cavity; and plugging said opening with a further material. In this method, the central pillar anchoring the membrane to the substrate of the capacitive pressure sensor is formed before the removal of the sacrificial material, which ensures that a stable and reproducible manufacturing process is obtained.

Preferably, the method further comprises depositing an etch stop layer over a region of the membrane material at least defining a membrane of the capacitive pressure sensor; covering the etch stop layer with the further material when plugging said opening; and selectively removing the further material from said region terminating on the etch stop layer, thereby forming a rim of the further material around said region. This ensures that a thin membrane, e.g. having a thickness in a range of 0.8-1.5 micron can be accurately formed as the membrane is thinned at the back end of the process. As explained above, the thus formed rim around the membrane can further assist in stabilizing the membrane during operation of the capacitive pressure sensor.

In a particularly preferred embodiment, said region laterally extends beyond the outer edge of the annulus of sacrificial material, for example by an amount of 2-5 microns, such that a clearance is formed between the membrane and the rim. As explained above, this assists in suppressing drift in the sensitivity of the capacitive pressure sensor over time due to contamination gathering on this clearance rather than on the outer perimeter of the membrane.

Yet more preferably, the method further comprises depositing a protective layer over the membrane material carrying the etch stop layer prior to opening the membrane material to expose said annulus. This reinforces the membrane layer by temporarily increasing its thickness with the protective layer, which protects the membrane layer from warping or buckling when the sacrificial material is released to form the cavity. This is particularly relevant for relatively thin membranes, e.g. membranes having a thickness or 2 micron or less, where the provision of such a protective layer can significantly improve the yield of the manufacturing process.

### BRIEF DESCRIPTION OF THE DRAWINGS

Embodiments of the invention are described in more detail and by way of non-limiting examples with reference to the accompanying drawings, wherein:
FIG. 1 schematically depicts a cross-sectional view of an ultrasound pressure sensor according to an embodiment;
FIG. 2 schematically depicts a method of manufacturing an ultrasound pressure sensor according to a preferred embodiment;
FIG. 3 schematically depicts a cross-sectional view of an ultrasound pressure sensor according to another embodiment;
FIG. 4 schematically depicts a cross-sectional view of an ultrasound pressure sensor according to yet another embodiment;
FIG. 5 depicts an electron microscope image of part of an ultrasound pressure sensor according to an embodiment;
FIG. 6 schematically depicts a medical instrument for insertion into a patient according to an example embodiment; and
FIG. 7 schematically depicts a cross-sectional view of an ultrasound transducer.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

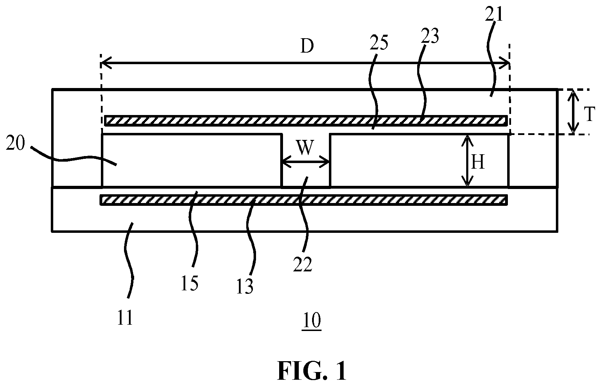

FIG. 1 schematically depicts a cross-sectional view of a capacitive pressure sensor 10 according to an embodiment of the present invention. The capacitive pressure sensor 10 maybe micro-machined e.g. maybe a CMUT. In at least some embodiments, the capacitive pressure sensor 10 is operable in a non-collapse mode as will be explained in further detail below. For the avoidance of doubt, a non-collapse mode is defined as a mode in which the flexible membrane 21 of the capacitive pressure sensor 10 does not contact the substrate 11 of the capacitive pressure sensor 10 during pressure sensing. The substrate 11 of the capacitive pressure sensor 10 typically is separated from the flexible membrane 21 by a cavity 20. In accordance with at least some embodiments of the present invention, the cavity 20 is an annular cavity around a central pillar 22 that permanently attaches a central region of the flexible membrane 21 to the substrate 11 of the ultrasound pressure sensor 10.

A first electrode 13 is present on the substrate 11 and is opposed by a second electrode 23 on the flexible membrane 21, which may be annular in shape in some embodiments such that the annular second electrode 23 aligns with the annular cavity 20. However, in FIG. 1 the second electrode 23 is a continuous or circular electrode. Such a continuous electrode provides more effective shielding of the first electrode 13 during use of the ultrasound pressure sensor 10, in particular where the first electrode 13 is used as the sensing electrode and the second electrode 23 is used as the reference or ground electrode, such that the first electrode 13 is effectively shielded from electrostatic interference from the medium by the second electrode 23. In order to prevent a short circuit between the first electrode 13 and the second electrode 23, the first electrode 13 maybe covered by a thin dielectric layer 15 and/or the second electrode 23 may be covered by a thin dielectric layer 25. Where the first electrode 13 is covered by the thin dielectric layer 15, the first electrode 13 maybe considered to be (embedded) in the substrate 11, and where the second electrode 23 is covered by the thin dielectric layer 25, the second electrode 23 may be considered to be (embedded) in the flexible membrane 21. It should be understood that in such embodiments, the thin dielectric layer 15 may be of a different material as the substrate 11 and the thin dielectric layer 25 maybe of a different material as the flexible membrane 21, i.e. these dielectric layers are not necessarily of the same material as the substrate 11 and at least part of the flexible membrane 21 respectively.

As will be readily understood by the skilled person, the first electrode 13 and the second electrode 23 define opposing plates of a capacitor separated by the annular cavity 20 (and the thin dielectric layer(s) 15 and/or 25 if present), with the capacitance of this capacitor being defined by the distance between these opposing electrodes. Therefore, as the membrane 21 is flexible, changes in pressure on the flexible membrane 21 causes the distance between these electrodes to change due to the changes in the degree of flexing or bowing of the flexible membrane 21. The associate change in capacitance of the capacitor may be measured by applying a potential difference across the opposing electrodes 13, 23 from which the pressure on the flexible membrane 21 may be derived. As this is well-known per se, this will not be further explained for the sake of brevity only.

For a capacitive pressure sensor 10 operable as an in-vivo pressure sensor, it is preferred that the flexible membrane 21 does not collapse onto the substrate 11 of the capacitive pressure sensor 10 during exposure to typical systolic blood pressures, e.g. up to or beyond 200 mm Hg in severe cases of hypertension in order to avoid the aforementioned hysteresis effects in the measurement of the patient's blood pressure with such a capacitive pressure sensor 10. As will be immediately understood by the skilled person, such a blood pressure is expressed relative to ambient pressure under which the blood pressure is determined, and typically expresses an excess to such an ambient pressure. Without wishing to be bound by theory, it is believed that such hysteresis effects may result from the flexible membrane 21 sticking to the substrate 11 during collapse, thus delaying release of the flexible membrane 21 upon a reduction of pressure, e.g. a drop in blood pressure, being exerted on the flexible membrane 21.

To this end, the capacitive pressure sensor 10 is dimensioned such that the (ambient) pressure P_{collapse} at which the flexible membrane 21 collapses onto the substrate 11 of the capacitive pressure sensor lies in the following range: 1.7 bar < P_{collapse} < 2.3 bar, with P = 0 bar corresponding to vacuum. If the collapse pressure of the flexible membrane 21 is 1.5 bar or less, there is an increasing risk that the flexible membrane 21 collapses onto the substrate 11 during use of the capacitive pressure sensor 10, whereas if the collapse pressure of the flexible membrane is 3.0 bar or more, the sensitivity of the capacitive pressure sensor 10 maybe insufficient. In an example embodiment, the collapse pressure P_{collapse} of the flexible membrane 21 is about 1.7 bar.

In order to achieve the desired collapse pressure of the flexible membrane 21, the flexible membrane 21 typically has an outer diameter D in a range of 20-200 µm (micron), preferably an outer diameter D in the range of 50-150 µm. The thickness T of the flexible membrane 21 preferably lies in a range of 0.8-2.0 µm when the flexible member 21 has the aforementioned outer diameter D. In order to prevent collapse of the flexible membrane 21 having the aforementioned dimensions onto the substrate 11 of the capacitive pressure sensor 10, the gap height H of the annular cavity 20 lies in a range of 50-500 nm, preferably in a range of 100-400 nm. If the gap height H is less than 50 nm, the collapse pressure of the flexible membrane 21 maybe below 1.5 bar, whereas if the gap height H is more than 500 nm, the sensitivity of the capacitive pressure sensor 10 maybe insufficient. The inner diameter of the flexible membrane 21, i.e. the width W of the central pillar 22 preferably is chosen such that 0.05D < W <0.3D in order to give the flexible membrane 21 its desired flexibility. Of course, as previously explained it should be understood that the aforementioned dimensions of the capacitive pressure sensor 10 are scalable such that other dimensions may be used without departing from the teachings of the present invention, e.g. based on the relationship P_{collapse} ∼ T³ * H/D⁴. What is more, depending on the application domain, other ratios of the aforementioned dimensions may be contemplated as for instance where the capacitive pressure sensor 10 is operable as an ultrasound transducer such that different collapse pressures for the flexible membrane 21 may be contemplated.

The ultrasound pressure sensor 10 maybe manufactured in any suitable manner, i.e. any manufacturing method in which the annular cavity 20 is defined by an annular portion of a sacrificial material on the substrate 11 on which the first electrode 13 and optional dielectric layer 15 is formed, after which the optional dielectric layer 25 and second electrode 23 may be formed over the resulting structure, followed by the formation of the flexible membrane 21, during which the formation of the central pillar 22 maybe realized and subsequent release of the sacrificial material to form the annular cavity 20 and sealing of the one or more openings through which the sacrificial material was released. The sealing material used to seal such one or more openings may form part of the thickness of the flexible membrane 21, in which case the flexible membrane 21 is only formed to its part of its target thickness upon the release of the sacrificial material, with the remaining thickness being provided by the deposition of the sealing material.

However, although this is a commonly deployed manufacturing strategy for such capacitive pressure sensors, a drawback of this strategy in particular in embodiments in which the capacitive pressure sensor 10 is to be operable as an in vivo pressure sensor is that upon release of sacrificial material to form the annular cavity 20 the flexible membrane 21 has a partial thickness of well below 1 µm. It has been found that where such capacitive pressure sensors 10 are formed in large numbers (tens of thousands) on a wafer, this leads to large variations in the collapse pressure between capacitive pressure sensors on such a wafer, which reduces the yield of the manufacturing process and increases post-processing checking as each capacitive pressure sensor must be checked to ensure that its collapse pressure (or collapse voltage for an ultrasound transducer) lies within the desired specifications.

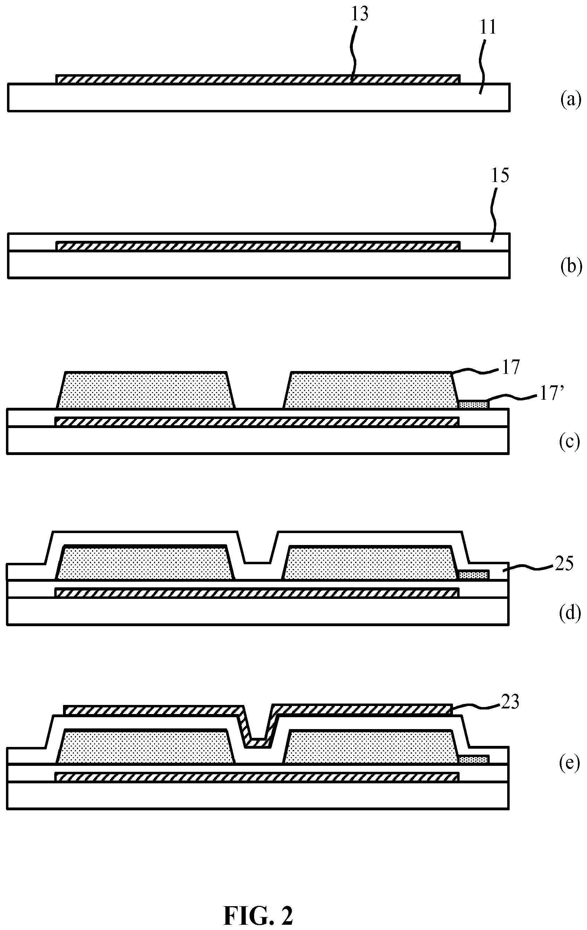

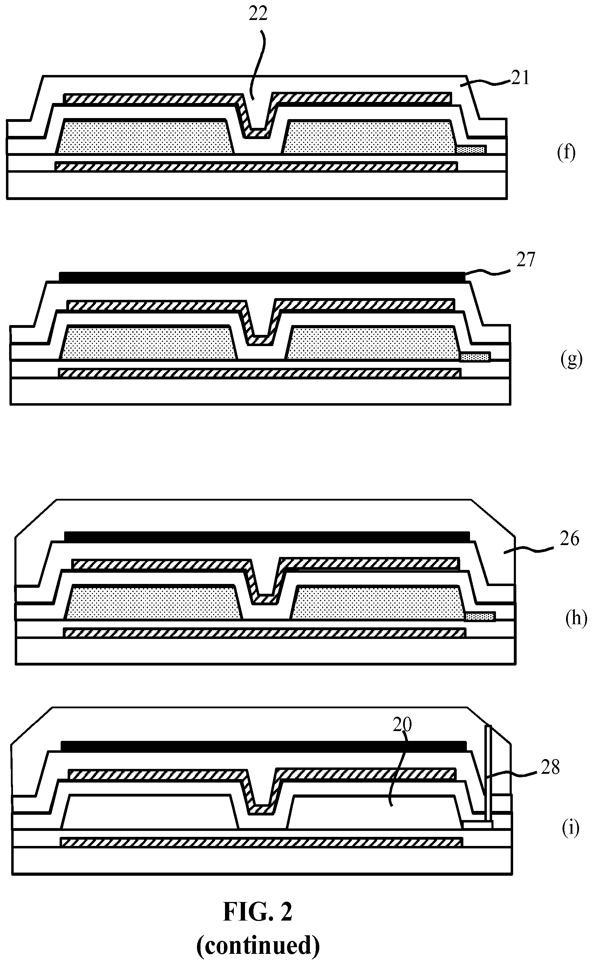

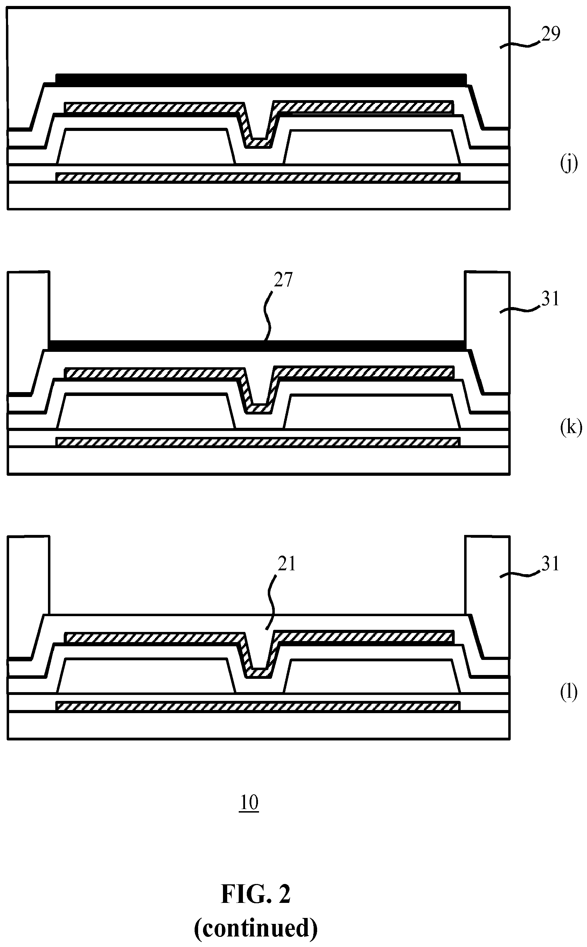

An important insight in the manufacturing of such capacitive pressure sensors (as well as of capacitive transducers), and in particular to drastically reduce variation in collapse pressure between such capacitive pressure sensor across a wafer on which the capacitive pressure sensor are formed, is that the flexible membrane 21 of the capacitive pressure sensor 10 is formed to its full thickness prior to the release of the sacrificial material in order to form the annular cavity 20 such that the flexible membrane 21 is more robust against warping or bowing during the release of the sacrificial material, which warping or bowing causes a change in the collapse pressure (or collapse voltage) of such a capacitive pressure sensor. This will be explained in further detail with the aid of FIG. 2, in which the various process steps of an example embodiment of this manufacturing process are schematically depicted.

In step (a), the first electrode 13 is formed on the substrate 11, which typically forms part of a wafer such as an ASIC wafer including circuit elements for controlling the capacitive pressure sensor 10. The provision of such wafers is well-known per se and is therefore not explained in further detail for the sake of brevity only. However, it is important to note that due to the presence of signal processing circuitry (not shown) in the substrate 11, the capacitive pressure sensor 10 can be effectively miniaturized, e.g. to overall dimensions of well below 100 microns. Such miniaturization may be further supported by the inclusion of circuitry that facilitates the communication of the sensor readings to a user console or the like to which a medical instrument including the capacitive pressure sensor 10 is connected over the power supply wires as a modulation of the power supply, such that a chip carrying the capacitive pressure sensor 10 only needs two power supply terminals as a dedicated sensor signal terminal may be omitted. It is noted that without the presence of such an ASIC in the substrate 11 it will not be possible to miniaturize the capacitive pressure sensor 10 to such an extent, given that for a capacitive pressure sensor 10 according to a typical embodiment of the present invention, the strength of the sensor signal lies in or below the pF domain, which signal is too weak to be successfully transported to a user console through wires extending through the medical instrument without some initial signal processing, e.g. amplification and/or digitization.

It should furthermore be understood that although step (a) depicts the formation of a single electrode 13, in reality many of such spatially separated electrodes are formed simultaneously on the wafer, which each electrode corresponding to a separate capacitive pressure sensor 10 to be formed thereon. The first electrode 13 may be made of any suitable electrically conductive material. The first electrode 13 may be made of a single layer of such materials or multiple layers of different electrode materials, e.g. to tune the conductive and stress properties of the first electrode 13. In an example embodiment, the first electrode 13 comprises a layer of AINd (aluminium/Neodymium) and a layer of WTi (tungsten/tin), which yields a particularly low-stress electrode.

In optional step (b), the surface of the substrate 11 carrying the first electrode 13 (i.e. the wafer surface) is covered by a thin layer of a dielectric material 15 to electrically insulate the first electrode 13. Any suitable dielectric material may be used for this layer, which may be deposited using any suitable deposition technique, e.g. atomic layer deposition (ALD) or (plasma-enhanced) chemical vapor deposition ((PE)CVD). Examples of such materials include silicon nitride (SiN) and silicon dioxide (SiO₂). In a particularly advantageous embodiment, a SiO₂ layer maybe formed by the deposition and subsequent chemical decomposition of TEOS, which leads to a high density dielectric layer having a very low defect density, i.e. few pinholes, thereby providing excellent electrical insulation.

In step (c), an annular portion 17 of a sacrificial material is formed, e.g. deposited, on the resultant structure. The annular portion 17 defines the annular cavity 20 to be formed and as such typically is formed to a height that equals the desired height of the annular cavity 20, e.g. a height in a range of 50-500 nm, such as a height in a range of 100-400 nm. One or more fingers 17' of the sacrificial material laterally extend from the annular portion 17, which are used to access and release the sacrificial material at a later stage in the manufacturing process as will be explained in more detail below. The sacrificial material preferably is AINd due to its ease of etching, with many etch recipes readily available for this material. However, alternative sacrificial materials such as molybdenum (Mo), an Al/Mo layer stack and AlCu may be contemplated as well, although the removal of such alternative materials is not as straightforward as the removal of AlNd.

In optional step (d), a further dielectric layer 25 is formed over the structure resulting after step (c). The same materials as previously described for the optional dielectric layer 15 may be used for the further dielectric layer 25, with TEOS again being particularly preferred. Thereafter, the second electrode 23 is formed in step (e), for which the materials as previously described for the first electrode 13 may be contemplated, such as a WTi/AINd layer stack in a particularly preferred embodiment. The WTi layer if present in either of the first electrode 13 and the second electrode 23 typically is arranged proximal to (i.e. facing) the annular cavity 20 in such a layer stack. As previously explained, the second electrode 23 preferably has a continuous or closed structure such as a circular shape, although alternative shapes, e.g. an annular second electrode 23 may also be contemplated. As shown in step (e), where such a closed second electrode 23 is formed, the central portion of the second electrode 23 may sag or dip into the base of the central pillar 22. This does not materially affect the performance of the capacitive pressure sensor 10.

In step (f), the flexible membrane 21 is deposited, e.g. using ALD or (PE)CVD, to its full target thickness using any suitable material or combination of materials in case the flexible membrane 21 is formed as a layer stack, such as SiN, SiO₂ and TEOS. SiN is particularly preferred for its mechanical properties. During this step, the formation of the central pillar 22 is also completed such that the center of the flexible membrane 21 is anchored onto the substrate 11 of the capacitive pressure sensor 10 as previously explained. In at least some embodiments, the thickness of the flexible membrane 21 ranges from 0.8-2.0 µm as previously explained.

In step (g), an etch stop layer 27 is deposited over a central region of the deposited membrane layer in step (f). The lateral dimensions of the etch stop layer 27 in a first set of embodiments are chosen such that the edge of the etch stop layer 27 aligns with the outer edge of the sacrificial material portion 17. However, in an alternative set of embodiments, the edge of the etch stop layer 27 lies outside this outer edge as will be explained in further detail below. The etch stop layer 27 may be made of any material that is resistant to the typical etch recipes used for etching silicon nitride or any other material used to seal or plug the one or more openings through which the sacrificial material portion 17 is released from underneath the flexible membrane 21 as will be described in more detail below. For example, the etch stop layer 27 maybe an Al/Mo alloy layer although other suitable etch stop materials will be immediately apparent to the skilled person.

In step (h), a protective layer 26 is formed over the structure resulting after step (g). This for example maybe a SiO₂ layer, a SiN layer or a layer (stack) formed of a combination of these materials. This protective layer is used to temporarily increase the thickness of the flexible membrane 21 such that upon release of the sacrificial material portion 17 to form the annular cavity 20, the flexible membrane 21 is less prone to warping or buckling. It should be understood that this strategy is not necessarily limited to the formation of capacitive pressure sensors or transducers having an annular cavity 20 but may be deployed in the manufacture of any type of capacitive pressure sensor or transducer having a relatively thin flexible membrane 21, e.g. a flexible membrane having a thickness of about 2 µm or less.

In step (i), one or more openings 28 are etched through the layer deposited to form the flexible membrane 21, e.g. using a suitable mask or the like as is well-known per se. The one or more openings 28 typically extend through this layer outside the area defining the annular cavity 20 of the capacitive pressure sensor 10 and extend to the fingers 17' of the sacrificial material. Alternatively, the opening 28 maybe formed in the central pillar 22 of the capacitive pressure sensor 10. Through these one or more openings 28, the sacrificial material portions 17 and 17' are removed using any suitable etch recipe in order to form the annular cavity 20 in between the substrate 11 and a flexible membrane 21 of the capacitive transducer 10.

In step (j), the one or more openings 28 are plugged with a sealing material 29, e.g. SiN or the like. The sealing material 29 is typically deposited over the full area of the wafer such that the previously deposited protective layer 26 is also covered by this sealing material. The sealing material 29 may be deposited in any suitable manner, e.g. using ALD or (PE)CVD byway of non-limiting example.

Next, in step (k), the sealing material 29 and protective layer 26 are selectively removed in a central region of the capacitive pressure sensor 10 using any suitable etch recipe or combination of etch recipes, with this etching step terminating on the etch stop layer 27. As is well-known per se, such a selective etch may be executed using a suitable mask or the like. In this step, the thickness of the flexible membrane 21 is reduced to its target thickness. The resultant structure of the capacitive pressure sensor 10 is characterized by the formation of a rim 31 around the flexible membrane, which may further stabilize the flexible membrane 21. In a top view, the flexible membrane 21 can be seen to be recessed within such rim 31 surrounding the flexible membrane 21. Although not shown in FIG. 2, such a rim 31 may further comprise an overhang over the flexible membrane 21 (i.e. an inverse L-shape in a cross-sectional view), which overhang may be formed when an anisotropic etch recipe is used to reduce the thickness of the flexible membrane as explained above.

Finally, in step (1) the etch stop layer 27 is removed from the flexible membrane 21 in any suitable manner, e.g. using a suitable etch recipe, after which the wafer may be diced to form the individual capacitive pressure sensors 10 or arrays of such pressure sensors. The aforementioned process steps have in common that they may all be performed at moderately low temperatures, i.e. below 400°C, thereby protecting the ASIC in the substrate 11 from destruction. A particular advantage of this manufacturing method is that the capacitive pressure sensor 10 can be kept very thin, thereby facilitating integration on very fine minimally invasive instruments, e.g. a catheter for insertion into the cardiovascular system of a patient. In some embodiments, the capacitive pressure sensor 10 may have a thickness of around 70 micron, which may be achieved by thinning the ASIC wafer (i.e. the substrate 11) onto which the sensor is formed using a sacrificial etch step for example.

Although not explicitly shown, in a further variation a protective layer may be formed, e.g. through coating, spraying, or the like, over the capacitive pressure sensors 10 prior to or after individualization. Such a protective layer may be made of a biocompatible electrically insulating material such as a silicone such as PDMS, parylene, polybutadiene rubber or foils such as a TPX or PET foil, or a combination of such materials to protect the capacitive pressure sensor 10 from fouling and/or to protect the patient from electrical shock.

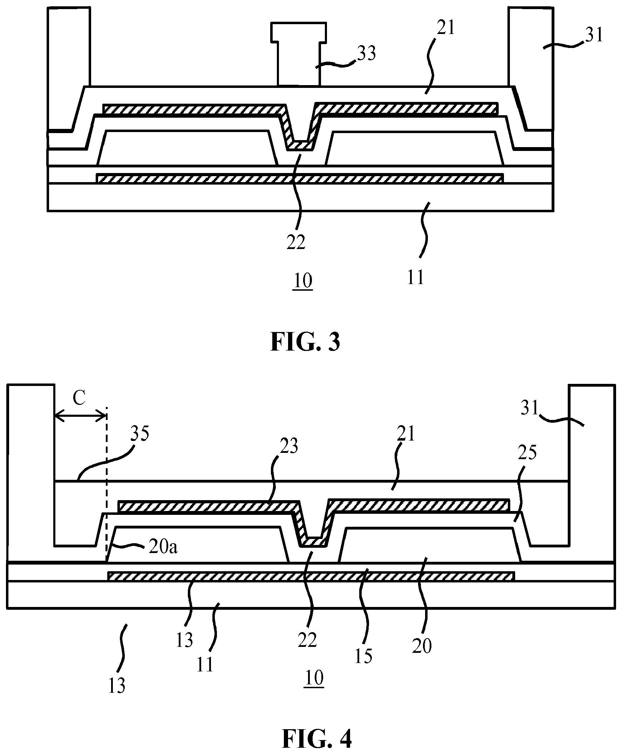

FIG. 3 shows a variation to the capacitive pressure sensor 10 as formed using the manufacturing process of FIG.2, as can be determined by the characteristic rim 31 around the flexible membrane 21. In this embodiment, the flexible membrane 21 further comprises a protrusion 33 extending from the central pillar 22 in a direction away from the floor or substrate 11 of the capacitive pressure sensor 10. Such a protrusion 33 may be formed when the annular portion 17 of the sacrificial material is released through an opening extending through the central pillar 22, after which this opening is sealed as explained above, which yields the characteristic T-shaped protrusion 33 on top of the central pillar 22.

FIG. 4 schematically depicts a preferred embodiment of the capacitive pressure sensor 10 of the present invention. In this embodiment, the rim 31 is laterally displaced from the outer edge 20a of the annular cavity 20 (i.e. from the flexible membrane 21) by a clearance 35 having a width C in a range of 2-5 micron, e.g. 4 micron. As will be readily understood, such a clearance 35 may be created by adjusting the dimensions of the etch stop layer 27 and the mask used in the etching step to reduce the thickness of the flexible membrane 21 as explained above in more detail with the aid of FIG. 2. It surprisingly has been found that the introduction of such a clearance 35 significantly improves the operational stability of the capacitive pressure sensor 10 over a prolonged period of time. For example, it was found that the introduction of the clearance 35 around the flexible membrane 21 significantly reduced the drift in sensitivity experienced by such a pressure sensor over time.

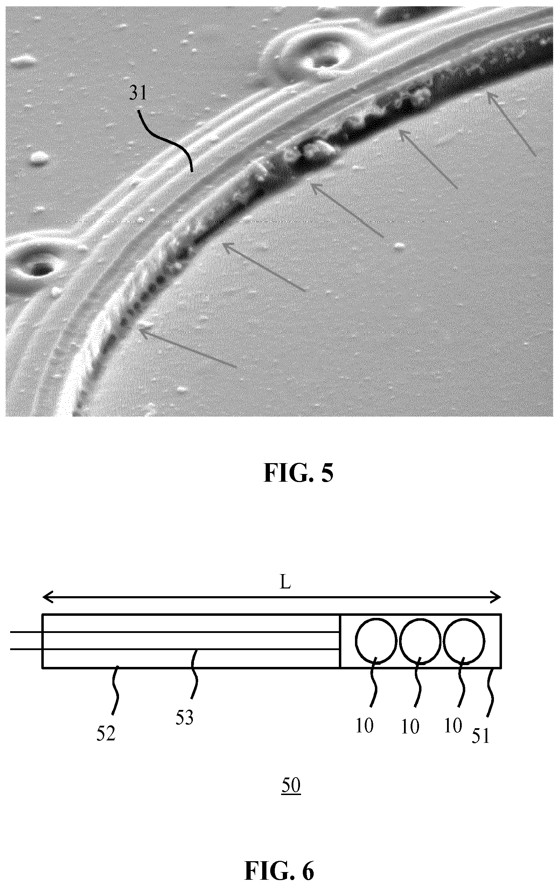

Without wishing to be bound by theory, it is believed that this can be explained by the fact that over time contamination accumulates on the exposed surface of the capacitive pressure sensor 10 during its use. As can be seen in FIG. 5, such contamination predominantly accumulates against the inner surface of the rim 31 as indicated by the arrows. Such contamination can include segmentation such as dirt particles or other solid matter present for example in a patient's blood that accumulates on the exposed surface of the capacitive pressure sensor 10. Where the rim 31 is aligned with the outer edge of the annular cavity 20, such contamination accumulates on the outer periphery of the flexible membrane 21, thereby altering the mass of the flexible membrane 21 and its flexibility (and resonance frequency) as a result. This causes a change in the sensitivity of the capacitive pressure sensor 10, thus leading to the aforementioned drift. The clearance 35 ensures that the bulk of such contamination accumulates outside the flexible membrane 21, such that the characteristics of the flexible membrane 21 remain largely unaffected, thereby significantly reducing the drift in the sensitivity of the capacitive pressure sensor 10. In experiments, it was found that the introduction of the clearance 35 around the flexible membrane 21 reduced sensor drift by at least 80% to less than 2 mmHg/h.

At this point it is noted that although the capacitive pressure sensor 10 preferably is formed in accordance with the above described manufacturing method, other manufacturing methods may also be contemplated. For example, the capacitive pressure sensor 10 may be formed using the well-known wafer bonding techniques. In such a wafer bonding technique, in which a membrane wafer is bonded onto a substrate wafer in which the cavity is etched into the substrate in order to form the capacitive micromachined element, as is described in more detail in WO2016/011000 A1 for example. It will be immediately understood by the skilled person that this process equally may be applied for the manufacture of the capacitive pressure sensor 10 according to embodiments of the present invention.

FIG. 6 schematically depicts a medical instrument 50 for insertion into a patient according to an example embodiment. The medical instrument 50 comprises an instrument tip 51 that is typically inserted into a patient during use of the medical instrument 50. The instrument tip 51 carries one or more capacitive pressure sensors 10 according to any embodiments of the present invention at its surface. In a preferred embodiment, the one or more capacitive pressure sensors 10 are operable to monitor local blood pressure within an artery or a vein of the patient. For example, such pressure sensors may be deployed to determine a gradient in the blood pressure of the patient between a first location and a second location within the artery or vein of the patient, which may be indicative of the presence of a narrowing anomaly such as a lesion or stenosis in between the first and second locations.

Preferably, the medical instrument 50 comprises a plurality of such capacitive pressure sensors 10 organized in an array along the elongation direction L of the medical instrument 50. In such an array, the capacitive pressure sensors 10 typically are spatially separated from each other in the elongation direction L such that the capacitive pressure sensors 10 may simultaneously determine the local blood pressure in a plurality of locations within the artery or vein of the patient with a stationary tip 51 such that the location of the anomaly can be associated with the position of the one or more capacitive pressure sensors 10 on the tip 51 across which the gradient in the blood pressure is observed such that upon determination of the position of the tip 51 within the vein of artery of the patient, the location of the anomaly can be accurately pinpointed. As is well-known per se, the position of the tip 51 within the vein or artery of the patient may be determined in any suitable manner, for example using imaging techniques such as X-ray, ultrasound, CT and so on.

Alternatively, the medical instrument 50 comprises a single capacitive pressure sensor 10 in which case local blood pressure within the vein or artery of the patient is periodically sampled whilst moving the tip 51 through the vein or artery such that the location of the anomaly may be directly associated with the position of the single pressure sensor on the tip 51 by determining the location of the tip 51 at which the gradient in this local pressure is observed, as this is the most likely location of the anomaly within the artery or vein.

The medical instrument 50 may further comprise a body 52 onto which the tip 51 is mounted through which wires 53 extend, e.g. bifilar wires within the body 52. As previously explained, in a preferred embodiment only two of such wires 53 are required, such that the sensor signal is added by the ASIC in the substrate 11 of the capacitive pressure sensor 10 to the power supplied over these wires. The wires 53 are typically connected to the respective electrodes 13, 23 of the one or more capacitive pressure sensors 10 and to connectors at a distal end of the invasive medical instrument 50 relative to the tip 51 such that the one or more capacitive pressure sensors 10 can be connected to a control module or user console through which the one or more capacitive pressure sensors 10 can be controlled. As such arrangements are well-known per se, they are not explained in further detail for the sake of brevity only. It suffices to say that any suitable arrangement for connecting the one or more capacitive pressure sensors 10 to such a control module may be used. The medical instrument 50 may take any suitable shape, such as a catheter or a guide wire. Due to the compact nature of the capacitive pressure sensor 10, i.e. having a height or thickness as little as 70 µm, the capacitive pressure sensor 10 according to embodiments of the present invention can be used on extremely thin invasive medical instruments, e.g. catheters that fit in a blood vessel having a diameter in a range of 0.5 -10 mm. For example, the capacitive pressure sensor 10 may be used on a FFR catheter for heart applications having a typical diameter of about 0.3 mm.

In the foregoing, the capacitive pressure sensor 10 advantageously comprised an annular cavity 20 around a central pillar 22 permanently anchoring the central portion of the flexible membrane 21 to the substrate 11 of the capacitive pressure sensor. However, it should be understood that certain aspects disclosed in the context of the present invention equally may be applied to other types of capacitive elements, e.g. capacitive transducers operable as ultrasound transducers and/or capacitive transducers having a circular rather than an annular cavity 20, i.e. capacitive transducers in which the central pillar 22 has been omitted from their design.

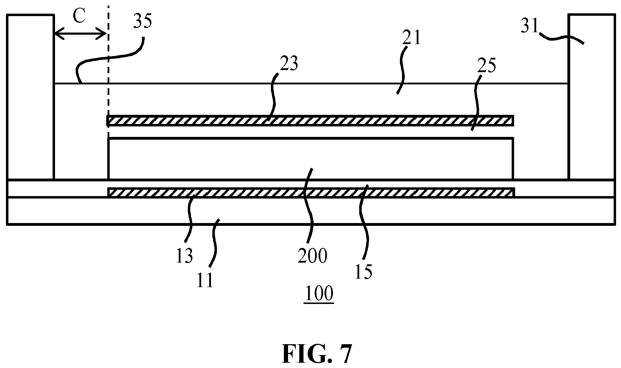

For example, as schematically depicted in FIG. 7, a capacitive element 100 such as a capacitive pressure sensor or a capacitive transducer may be manufactured in accordance with the manufacturing method explained in the foregoing with the aid of FIG. 2, in which the annular cavity 20 is replaced by a circular cavity 200. As will be readily understood by the skilled person, this may be achieved by altering the shape of the portion 17 of the sacrificial material deposited on the substrate 11 (i.e. the wafer) to a circular rather than an annular shape. The manufacture of such a capacitive element 100 in this manner also benefits from excellent yield due to the inclusion of the protective layer 26 in the manufacturing process, which temporarily increases the thickness of the flexible membrane 21 and protects the respective flexible membranes across the wafer from warping or buckling during the release of the sacrificial material, thereby reducing variations in the membrane properties, e.g. collapse voltage, across the wafer. This is particularly suited for the production of capacitive transducers 100, e.g. CMUTs, having a relatively thin membrane, e.g. a membrane thickness T of 2 µm or less spanning a relatively wide cavity 200, e.g. having a diameter D of 100 µm or more, i.e., capacitive transducers 100 having a ratio of D/T of 50 or more.

Also, the provision of the clearance 35 in between the rim 31 and the outer edge of the flexible membrane 21 (i.e. the outer edge of the circular cavity 200) improves the performance of such a capacitive element 100 in that the performance of such a capacitive element 100 is subject to smaller variations in its performance over time due to the fact that any contamination that accumulates on the exposed surface of the capacitive transducer 100 accumulates on the clearance 35 against the rim 31 rather than on the outer regions of the flexible membrane 21, such that the operational parameters, e.g. resonance frequency, of the flexible membrane 21 are not substantially altered by the collection of such contamination. As will be readily understood by the skilled person, the provision of the clearance 35 can improve the performance of any capacitive element such as a capacitive pressure sensor or capacitive transducer regardless of the shape of its cavity.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A capacitive pressure sensor (10) comprising:
a membrane (21) including a second electrode (23) spatially separated by a cavity (20) from a substrate (11) including a first electrode (13) opposing the second electrode; and
a central pillar (22) extending from the membrane to the substrate such that the cavity is an annular cavity enveloping said central pillar.

2. The capacitive pressure sensor (10) of claim 1, wherein the cavity (20) has an inner edge defined by the central pillar (22) and an opposing outer edge (20a), said inner edge and opposing outer edge having substantially the same height.

3. The capacitive pressure sensor (10) of claim 1, wherein the second electrode (23) has a closed structure.

4. The capacitive pressure sensor (10) of any of claims 1-3, wherein the membrane (21) has a thickness (T) in a range of 0.8-2.0 micron.

5. The capacitive pressure sensor (10) of claim 4, further comprising a rim (31) surrounding the membrane (21), said rim having a further thickness larger than the membrane thickness.

6. The capacitive pressure sensor (10) of claim 5, wherein the rim (31) is laterally displaced relative to the outer edge (20a) of the annular cavity (20) such that the rim is further removed from the central pillar (22) than said outer edge, preferably wherein the rim is laterally displaced relative to said outer edge by a distance in a range of 2-5 micron.

7. The capacitive pressure sensor (10) of any of claims 1-6, wherein the membrane (21) has a diameter (D) in a range of 20-200 micron.

8. The capacitive pressure sensor (10) of any of claims 1-6, wherein the annular cavity (20) has a height (H) in a range of 50-500 nm, preferably in a range of 100-400 nm.

9. The capacitive pressure sensor of any of claims 1-8, wherein the substrate (11) further comprises:
a circuit arrangement adapted to process sensor signals generated with the capacitive pressure sensor; and
a pair of terminals conductively coupled to the circuit arrangement for receiving power supply wires.

10. A medical instrument (50) for insertion into a patient having an instrument tip (51) comprising the capacitive pressure sensor (10) of any of claims 1-9.

11. The medical instrument (50) of claim 10, wherein the medical instrument comprises a plurality of said capacitive pressure sensors (10) distributed along an elongation direction (L) of the invasive medical instrument.

12. A method for manufacturing a capacitive pressure sensor (10), comprising:
providing a substrate (11);
forming a first electrode (13) on said substrate;
depositing an annulus (17) of a sacrificial material on the substrate;
forming a second electrode (23) on said annulus;
depositing a membrane material (21) over the annulus, thereby filling the central cavity of the annulus;
opening (28) the membrane material to expose said annulus;
removing the sacrificial material through said opening to form an annular cavity (20); and
plugging said opening with a further material (29).

13. The method of claim 12, further comprising:
depositing an etch stop layer (27) over a region of the membrane material at least defining a membrane (21) of the capacitive transducer (10);
covering the etch stop layer with the further material (29) when plugging said opening; and
selectively removing the further material from said region terminating on the etch stop layer, thereby forming a rim (31) of the further material around said region.

14. The method of claim 13, wherein said region laterally extends beyond the outer edge of the annulus of sacrificial material.

15. The method of claim 13 or 14, further comprising depositing a protective layer (26) over the membrane material carrying the etch stop layer (27) prior to opening the membrane material to expose said annulus.
